# EUROPEAN PATENT APPLICATION

(11) **EP 4 613 159 A1**
(43) Date of publication of application: **10.09.2025**
(21) Application number: 23903860.7
(22) Date of filing: 04.12.2023
(51) Int. Cl.: A47K 7/04, A45D 44/02

(54) **HOME SKIN CLEANSING DEVICE HAVING ELECTRIC CLEANSING FUNCTION**

(30) Priority: 12.12.2022 KR 20220172902
(71) Applicant: APR CO., LTD., Seoul 05551 (KR)
(72) Inventor: JI, Jong Chul, Seoul 05551 (KR); LEE, Gyoun Jung, Seoul 05551 (KR); PARK, Seung Woo, Seoul 05551 (KR)
(74) Representative: Henkel & Partner mbB
(86) International application number: PCT/KR2023/019758
(87) International publication number: WO 2024/128657

(57) **Abstract**

Disclosed is a home skin cleansing device having both an electric skin cleansing function and a skin vibration function by means of supply of a galvanic current. The home skin cleansing device comprises: a vibration plate 20 which contacts the skin of a user to transmit vibration; a vibration generation unit 22 which generates vibration to vibrate the vibration plate 20; a housing 10 having the vibration plate 20 and the vibration generation unit 22 mounted on one end thereof and a handle 30 mounted on the other end; and a galvanic current supply unit 50 which is provided inside the housing 10 and receives a direct current voltage and supplies a galvanic current of uniform current intensity to the skin of the user through a negative electrode 42 and a positive electrode 44, wherein the negative electrode 42 is mounted on one end of the vibration plate 20, and the positive electrode 44 is mounted on the handle 30. (Representative Drawing: FIG. 3)

## Description

### [Technical Field]

The present disclosure relates to a home skin cleansing device having an electric cleansing function, and more particularly, to a home skin cleansing device having both an electric cleansing function for the skin by supply of galvanic current and a vibration function for the skin.

### [Background Art]

Skin care refers to actions of applying cosmetics or cleansing products to the skin, receiving a skin massage, or removing foreign substances from the skin, in order to keep the skin clean and beautiful. For skin care, recently, a method using a skin cleansing device having a brush that rotates or vibrates has been used to uniformly apply a cleansing product to the skin or to cleanse the skin. For example, Korean Patent Application Publication No. 10-2017-0099518 and Korean Utility Model Registration No. 20-0489176 disclose a skin cleansing device for cleansing or massaging the skin by rotating a contact unit equipped with a brush that comes into contact with the skin. In addition, Korean Patent Registration No. 10-2324257 discloses a body cleansing device that vibrates a brush unit using a vibration motor or vibrates an ultrasonic head using an ultrasonic vibrator to provide ultrasonic vibration to a user.

Meanwhile, in the process of performing the skin's metabolism, waste substances are continuously generated and discharged, and thus it is necessary to appropriately remove the waste substances to keep the skin clean. Recently, galvanic beauty devices have been developed to enhance skin health by applying galvanic electrical stimulation to the skin, promoting blood circulation and expanding pores to facilitate the separation of waste substances, and increasing the absorption effect of various aqueous cosmetic compositions, including liquid-phase or viscous formulations, containing beneficial ingredients into the skin. The galvanic beauty device refers to a device that brings a positive electrode and a negative electrode into contact with the skin to form a closed circuit, and selectively moves cations or anions depending on the polarity of the electrical stimulation, thereby allowing various skin lotions to be absorbed into the skin or separating and removing waste substances remaining on the skin. For example, by iontophoresis using a galvanic current, an anionic substance may be pushed out from the negative electrode of the galvanic current generator to induce a saponification process caused by an alkaline reaction, thereby achieving effects such as sebum decomposition and skin softening. The iontophoresis technique for delivering active ingredients using galvanic current is disclosed in Korean Patent Application Publication No. 10-2012-0091335, Korean

Patent Registration No. 10-2137437, U.S. Patent Application Publication No. US 2006-0015052, and Korean Patent Registration No. 10-1653292. However, such electric cleansing or iontophoresis of the skin using galvanic current has a disadvantage in that it generally requires treatment by a separate specialist or the use of a separate dedicated galvanic beauty device.

### [Prior Art Documents]

(Patent Document 1) Korean Patent Application Publication No. 10-2012-0091335
(Patent Document 2) Korean Patent Registration No. 10-2137437
(Patent Document 3) U.S. Patent Application Publication No. US 2006-0015052
(Patent Document 4) Korean Patent Registration No. 10-1653292

### [Disclosure]

### [Technical Problem]

An object of the present disclosure is to provide a home skin cleansing device having both an electric skin cleansing function by supply of galvanic current and a skin vibration function.

Another object of the present disclosure is to provide a home skin cleansing device in which electrodes for supplying galvanic current and a vibration transmission device for the skin are efficiently arranged.

### [Technical Solution]

To achieve the above object, the present disclosure provides a home skin cleansing device comprising: a vibration plate 20 configured to contact the skin of a user and transmit vibration; a vibration generation unit 22 configured to generate vibration to vibrate the vibration plate 20; a housing 10 having the vibration plate 20 and the vibration generation unit 22 mounted on one end thereof and a handle 30 mounted on the other end; and a galvanic current supply unit 50 provided inside the housing 10, configured to receive a direct current voltage and supply a galvanic current of uniform current intensity to the skin of the user through a negative electrode 42 and a positive electrode 44, wherein the negative electrode 42 is mounted on one end of the vibration plate 20, and the positive electrode 44 is mounted on the handle 30.

### [Advantageous Effects]

The home skin cleansing device according to the present disclosure has both an electric skin cleansing function by supply of galvanic current and a skin vibration function, and electrodes for supplying the galvanic current and a vibration transmission device for the skin are efficiently arranged.

### [Brief Description of Drawings]

FIG. 1 and FIG. 2 are a perspective view of an exterior and a partial internal configuration view, respectively, of a home skin cleansing device having an electric skin cleansing function according to an embodiment of the present disclosure.
FIG. 3 is a perspective view of a home skin cleansing device having an electric skin cleansing function according to another embodiment of the present disclosure.
FIG. 4 is a view showing an example of a galvanic current supply unit 50 that may be used in the home skin cleansing device according to the present disclosure.
FIG. 5 is a view showing another example of the galvanic current supply unit 50 that may be used in the home skin cleansing device according to the present disclosure.
FIG. 6 is a view showing an example of a galvanic current waveform supplied from the galvanic current supply unit 50 of the home skin cleansing device according to the present disclosure.

### [Best Mode]

Hereinafter, the present disclosure will be described in detail with reference to the accompanying drawings. In the description of the present disclosure, detailed descriptions of well-known functions or configurations that are universally used will be omitted.

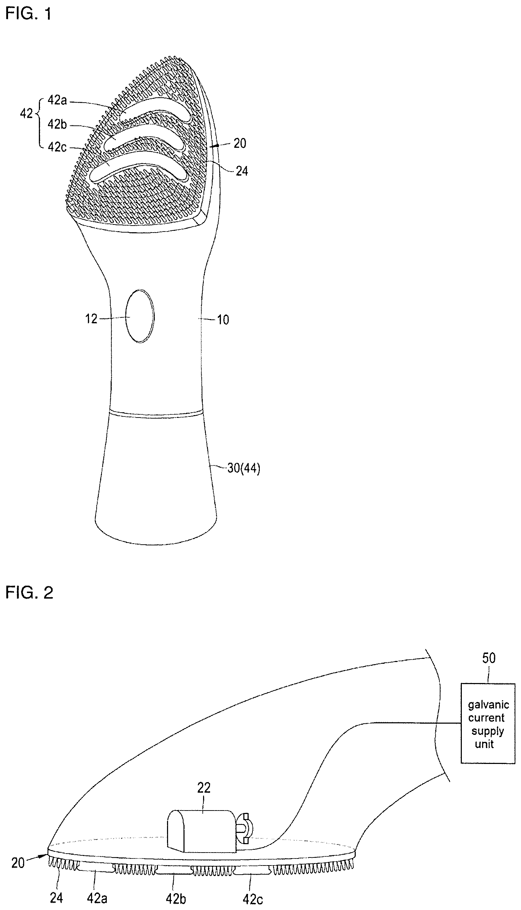

FIG. 1 and FIG. 2 are a perspective view of the exterior and a partial internal configuration view, respectively, of a home skin cleansing device having an electric cleansing function according to an embodiment of the present disclosure. As shown in FIGS. 1 and 2, the home skin cleansing device according to the present disclosure includes a housing 10, a vibration plate 20, a vibration generation unit 22, a handle 30, a galvanic current supply unit 50, a negative electrode 42, and a positive electrode 44.

In the home skin cleansing device of the present disclosure, the housing 10 may be provided in a substantially cylindrical shape so that a user can hold one end of the skin cleansing device with a hand, with the vibration plate 20 and the vibration generation unit 22 mounted on one end, and the handle 30 mounted on the other end, and the galvanic current supply unit 50 provided inside. The housing 10 may be made of a hard synthetic resin, or may be made of a synthetic resin having a predetermined elasticity, for example, silicone or rubber. If the side of the housing 10 coupled to the vibration plate 20 is made of a synthetic resin having a predetermined elasticity, the housing 10 may preferably absorb or buffer the vibration of the vibration plate 20. As shown in FIGS. 1 and 2, it is preferable that the vibration plate 20 and the handle 30 are respectively formed on opposite ends of the housing 10 so that they are not adjacent to each other. If the vibration plate 20 and the handle 30 are spaced apart as described above, it is possible to prevent the hand of the user gripping the handle 30 from directly contacting the vibration plate 20, thereby preventing the vibration of the vibration plate 20 or the current of the negative electrode 42 from being directly applied to the user's hand.

The vibration generation unit 22 generates vibration to vibrate the vibration plate 20, and the vibration plate 20 contacts the skin of the user and transmits the vibration. The vibration generation unit 22 may generate ultrasonic vibration or mechanical vibration. The ultrasonic vibration may be generated using a piezoelectric element or the like, and the mechanical vibration may be generated by a vibration motor or by an interaction between a coil and a magnet. According to a vibration control signal applied to the vibration generation unit 22, the piezoelectric element, vibration motor, or coil/magnet of the vibration generation unit 22 vibrates, and thus the vibration pattern of the vibration generation unit 22 and the vibration plate 20 may be adjusted by controlling the input pattern, frequency, or intensity of the vibration control signal. In one embodiment of the present disclosure, the vibration generation unit 22 may include a vibration motor, and the vibration plate 20 may vibrate in a direction perpendicular to the surface of the vibration plate 20 by rotation of the vibration motor. In another embodiment of the present disclosure, the vibration generation unit 22 may include an ultrasonic generator for generating ultrasonic waves, and the vibration plate 20, serving as an ultrasonic head, may perform ultrasonic vibration in a direction perpendicular to the surface of the vibration plate 20 by oscillation of the ultrasonic generator. On the surface of the vibration plate 20, a skin cleansing unit 24, which contacts the skin of the user to massage or cleanse the skin by friction, may be formed, and the skin cleansing unit 24 may include, for example, a brush, a sponge, a cleansing towel, a cleansing pad, or a protrusion. The vibration plate 20 may have its edge fixed to the end of the lower surface of the housing 10 so as to cover one end of the housing 10. The coupling between the vibration plate 20 and the housing 10 may be performed by general adhesive bonding, elastic adhesive bonding using silicone or the like, mechanical fitting, screw coupling, or the like.

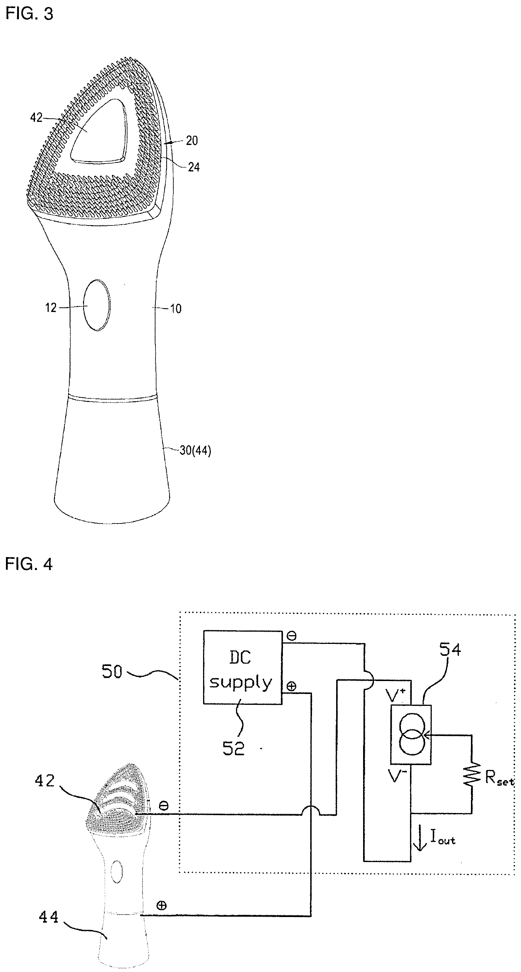

As shown in FIGS. 1 and 2, the negative electrode 42 may include a plurality of separated negative electrodes 42a, 42b, and 42c. When the negative electrode 42 is composed of a plurality of separated negative electrodes 42a, 42b, and 42c as described above, the galvanic current may be more efficiently supplied to a wider area of the skin of the user. FIG. 3 is a perspective view of a home skin cleansing device having an electric cleansing function according to another embodiment of the present disclosure. The skin cleansing device according to the present embodiment has the same structure as the embodiment shown in FIGS. 1 and 2, except for the arrangement structure of the negative electrode 42. As shown in FIG. 3, the negative electrode 42 according to the present embodiment has a plate shape formed on the surface of the vibration plate 20. When the negative electrode 42 has a plate shape as described above, the vibration of the vibration plate 20 and the galvanic current can be more efficiently and simultaneously transmitted to the skin of the user through the negative electrode 42. In this case, since the negative electrode 42 also serves as a vibration surface, it is preferable that the negative electrode 42 has a predetermined surface area, for example, an area of 20 to 40% of the surface area of the vibration plate 20.

The galvanic current supply unit 50, which is a constant current source, receives a direct current voltage and supplies a galvanic current of uniform current intensity to the skin of the user through the negative electrode 42 and the positive electrode 44. The galvanic current refers to a microcurrent used, by iontophoresis, to cause various aqueous cosmetic compositions to penetrate into the skin and be absorbed, or to separate and remove waste substances remaining on the skin. The negative electrode 42 is mounted on one end of the vibration plate 20, and the positive electrode 44 is mounted on the handle 30. Preferably, the handle 30 may be made of a conductive material and perform the role of the positive electrode 44. When the user grips the handle 30 of the home skin cleansing device according to the present disclosure and brings the vibration plate 20 into contact with the skin to be cleansed, the galvanic current generated by the galvanic current supply unit 50 flows through a closed circuit composed of the negative electrode 42, the skin of the user, the hand of the user, and the positive electrode 44, and is delivered to the skin of the user. In the home skin cleansing device according to the present disclosure, since the positive electrode 44 is located at one end of the skin cleansing device gripped by the user, and the cleansing surface in contact with the skin, that is, the vibration plate 20, has the negative electrode 42 located thereon, the galvanic current is supplied to the skin while the vibration function of the vibration cleansing device is performed.

FIG. 4 is a view showing an example of a galvanic current supply unit 50 that may be used in the home skin cleansing device according to the present disclosure. As shown in FIG. 4, the galvanic current supply unit 50 includes a DC supply 52 for supplying a direct current voltage, a constant current source 54 which receives the direct current voltage from the DC supply 52 and outputs a voltage of a constant current value, and a load resistor Rₛₑₜ for adjusting the output current value of the constant current source 54. The constant current source 54 is a 3-terminal adjustable current source that outputs a constant current value depending on the resistance of the load resistor Rₛₑₜ, and a general LM134/LM234/LM334 device (Texas Instruments, USA) may be used. For example, in the galvanic current supply unit 50 shown in FIG. 4, the output current lout can be calculated as lout = 67.8 mV/Rₛₑₜ.

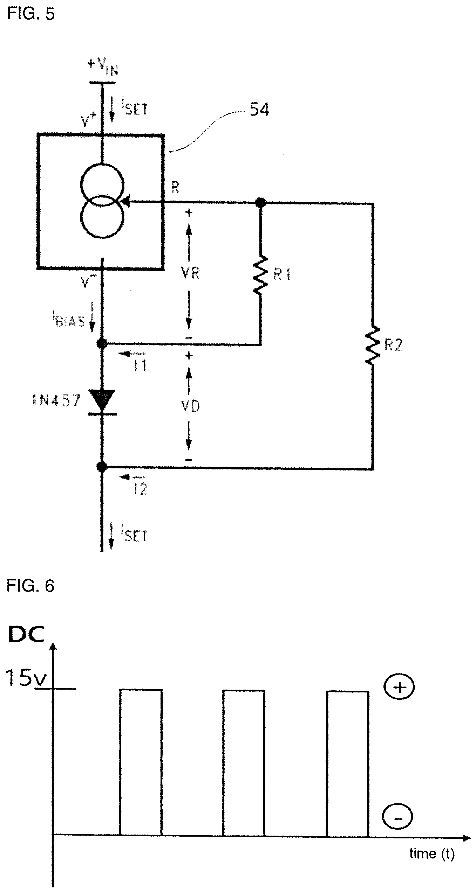

FIG. 5 is a view showing another example of the galvanic current supply unit 50 that may be used in the home skin cleansing device according to the present disclosure. In the example shown in FIG. 5, the DC supply 52 is omitted from the drawing, and an additional resistor R₂ and a diode 1N457 are added to the external circuit of the constant current source 54 as shown in FIG. 4, in addition to the load resistor R₁ = Rₛₑₜ, in order to eliminate the temperature dependency of the output current from the galvanic current supply unit 50.

The home skin cleansing device according to the present disclosure may supply a stable high-quality constant current regardless of skin condition by using the galvanic current supply unit 50 as shown in FIGS. 4 and 5. FIG. 6 is a view showing an example of a galvanic current waveform supplied from the galvanic current supply unit 50 of the home skin cleansing device according to the present disclosure. As shown in FIG. 6, the home skin cleansing device according to the present disclosure may supply, to the skin of the user, a galvanic current waveform in the form of a pulse of 15 V or more using a current of 500 µA, which is higher than that of conventional galvanic beauty devices that use a current of 100 µA.

When the user grips the handle 30 of the home skin cleansing device according to the present disclosure and brings the vibration plate 20 of the skin cleansing device into contact with the skin of the user to be cleansed, and then operates the skin cleansing device using a wireless controller or an operation switch 12 mounted on the skin cleansing device, the galvanic current generated by the galvanic current supply unit 50 flows through a closed circuit composed of the negative electrode 42, the skin of the user, the hand of the user, and the positive electrode 44, and is delivered to the skin of the user. In addition, simultaneously, the vibration generated by the vibration generation unit 22 vibrates the vibration plate 20, thereby transmitting the vibration to the skin of the user and performing skin care. According to the home skin cleansing device of the present disclosure, the electric cleansing function for the skin may be implemented through the negative electrode 42 and the positive electrode 44 mounted on the home skin cleansing device, along with the provision of vibration by the vibration plate 20.

Although the present disclosure has been described above with reference to the accompanying drawings and exemplary embodiments, the present disclosure is not limited to the matters shown in the drawings and the above-described embodiments. In the following claims, reference numerals are given for convenience of understanding, but the scope of rights of the claims is not limited to the reference numerals or the content shown in the drawings, and should be interpreted broadly to include modifications, equivalent structures, and functions of the exemplary embodiments.

### [Industrial Applicability]

The present disclosure relates to a home skin cleansing device having an electric cleansing function.

## Claims

1. A home skin cleansing device comprising:
a vibration plate 20 configured to contact a skin of a user and transmit vibration;
a vibration generation unit 22 configured to generate vibration to vibrate the vibration plate 20;
a housing 10 having the vibration plate 20 and the vibration generation unit 22 mounted on one end thereof and a handle 30 mounted on the other end; and
a galvanic current supply unit 50 provided inside the housing 10, configured to receive a direct current voltage and supply a galvanic current of uniform current intensity to the skin of the user through a negative electrode 42 and a positive electrode 44,
wherein the negative electrode 42 is mounted on one end of the vibration plate 20, and the positive electrode 44 is mounted on the handle 30.

2. The home skin cleansing device according to claim 1, wherein the vibration plate 20 and the handle 30 are respectively formed at opposite ends of the housing 10 so as not to be in contact with each other.

3. The home skin cleansing device according to claim 1, wherein the negative electrode 42 includes a plurality of separated negative electrodes 42a, 42b, and 42c.

4. The home skin cleansing device according to claim 1, wherein the negative electrode 42 has a plate shape formed on the surface of the vibration plate 20.

5. The home skin cleansing device according to claim 1, wherein the galvanic current supply unit 50 includes: a DC supply 52 configured to supply a direct current voltage; a constant current source 54 configured to receive the direct current voltage from the DC supply 52 and output a voltage of a constant current value; and a load resistor Rₛₑₜ configured to adjust an output current value of the constant current source 54.
